(19) Europäisches Patentamt
European Patent Office
Office européen des brevets

(11) Numéro de publication : **0 646 583 A1**

(12) **DEMANDE DE BREVET EUROPEEN**

(21) Numéro de dépôt : **94402114.6**

(22) Date de dépôt : **23.09.94**

(51) Int. Cl.$^6$ : **C07D 471/06,** C07D 498/06, C07D 487/06, A61K 31/445, A61K 31/535, A61K 31/55, // (C07D471/06, 235:00, 221:00), (C07D498/06, 265:00, 235:00), (C07D487/06, 243:00, 235:00), (C07D487/06, 241:00, 235:00)

(30) Priorité : **04.10.93 FR 9311771**

(43) Date de publication de la demande :
**05.04.95 Bulletin 95/14**

(84) Etats contractants désignés :
**AT BE CH DE DK ES FR GB GR IE IT LI LU MC NL PT SE**

(71) Demandeur : **SYNTHELABO**
**22, Avenue Galilée**
**F-92350 Le Plessis Robinson (FR)**

(72) Inventeur : **Jegham, Samir**
**65 rue du Lieutenant Colonel Prudhon**
**F-95100 Argenteuil (FR)**
Inventeur : **Defosse, Gérard**
**29 rue de Tolbiac**
**F-75013 Paris (FR)**
Inventeur : **Purcell, Thomas Andrew**
**47bis rue de la Millière Les Mesnuls**
**F-78490 Montfort l'Amaury (FR)**
Inventeur : **Even, Luc**
**172 avenue de Choisy**
**F-75013 Paris (FR)**

(74) Mandataire : **Thouret-Lemaitre, Elisabeth et al**
**SYNTHELABO,**
**Service Brevets,**
**B.P. 72**
**F-92352 Le Plessis-Robinson Cédex (FR)**

(54) **Dérivés d'imidazol-4-yl-pipéridine, leur préparation et leur application en thérapeutique.**

(57) Composés répondant à la formule (I)

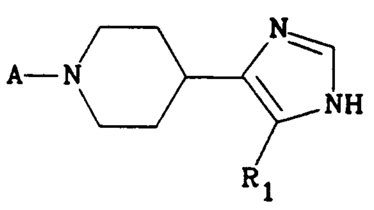

(I)

dans laquelle
$R_1$ représente soit un atome d'hydrogène, soit un groupe ($C_1$-$C_6$)alkyle droit ou ramifié et
A représente soit un groupe 5,6-dihydro-4*H*-imidazo[4,5,1-*ij*] quinoléin-2-yle, soit un groupe 4,5-dihydroimidazo[1,5,4-*de*] [1,4]benzoxazin-2-yle, soit un groupe 4-méthyl-4,5-dihydroimidazo[1,5,4-de][1,4]benzoxazin-2-yle, soit un groupe 4-phényl-4,5-dihydroimidazo[1,5,4-*de*][1,4]benzoxazin-2-yle, soit un groupe 4-(phénylméthyl)-4,5-dihydroimidazo[1,5,4-*de*][1,4] benzoxazin-2-yle, soit un groupe 5-méthyl-4,5-dihydroimidazo[1,5,4-*de*] [1,4]benzoxazin-2-yle, soit un groupe 5,6-dihydro-4*H*-imidazo [1,5,4-*de*]quinazolin-2-yle, soit un groupe 6-oxo-5,6-dihydro-4*H*-imidazo[4,5,1-*ij*]quinoléin-2-yle, soit un groupe 5-méthyl-4,5,6,7-tétrahydroimidazo[4,5,1-*jk*][1,4] benzodiazépin-2-yle éventuellement substitué en 6 par un groupe phénylméthyle ainsi que leurs sels d'additions aux acides pharmaceutiquement acceptables. Les composés de l'invention sont des ligands des récepteurs sérotoninergiques de types 5-$HT_3$ et 5-$HT_4$.

EP 0 646 583 A1

Jouve, 18, rue Saint-Denis, 75001 PARIS

La présente invention a pour objet des dérivés d'imidazol-4-yl-pipéridine, leur préparation et leur application en thérapeutique.

Les composés de l'invention répondent à la formule (I)

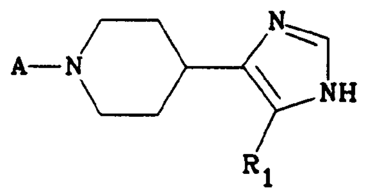

(I)

dans laquelle

$R_1$ représente soit un atome d'hydrogène, soit un groupe $(C_1-C_4)$alkyle droit ou ramifié et

A représente soit un groupe 5,6-dihydro-4*H*-imidazo[4,5,1-*ij*] quinoléin-2-yle, soit un groupe 4,5-dihydroimidazo[1,5,4-*de*] [1,4]benzoxazin-2-yle, soit un groupe 4-méthyl-4,5-dihydro-imidazo[1,5,4-*de*][1,4]benzoxazin-2-yle, soit un groupe 4-phényl-4,5-dihydroimidazo[1,5,4-*de*][1,4]benzoxazin-2-yle, soit un groupe 4-(phénylméthyl)-4,5-dihydroimidazo[1,5,4-de] [1,4]benzoxazin-2-yle, soit un groupe 5-méthyl-4,5-dihydroimidazo[1,5,4-*de*][1,4]benzoxazin-2-yle, soit un groupe 5,6-dihydro-*4H*-imidazo[1,5,4-*de*]quinazolin-2-yle, soit un groupe 6-oxo-5,6-dihydro-4*H*-imidazo[4,5,1-*ij*]quinoléin-2-yle, soit un groupe 5-méthyl-4,5,6,7-tétrahydroimidazo[4,5,1-*jk*] [1,4]benzodiazépin-2-yle éventuellement substitué en 6 par un groupe phénylméthyle.

Les composés de l'invention peuvent se présenter à l'état de bases libres ou de sels d'addition à des acides pharmaceutiquement acceptables.

Les composés dont la formule est une forme mésomère de la formule (I) font partie de l'invention. Certains composés de l'invention possèdent un carbone asymétrique et les isomères font également partie de l'invention.

Des composés de formule (I) dans laquelle A représente un groupe benzimidazole sont décrits dans la demande de brevet européen EP 0507650.

Conformément à l'invention, on peut préparer les composés de formule (I) selon le procédé illustré dans le schéma 1.

On fait réagir à chaud dans un solvant tel que l'alcool isoamylique un dérivé de formule (II) dans laquelle Z représente un atome d'halogène, de préférence un atome de chlore et A est tel que défini précédemment avec une pipéridine de formule (III) dans laquelle $R_1$ est tel que

Schéma 1

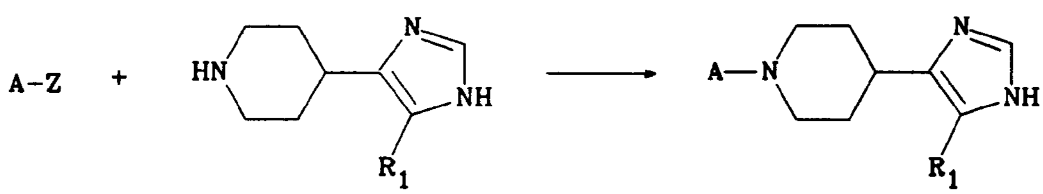

(II) (III) (I)

défini précédemment.

Les composés de départ sont décrits dans la littérature ou peuvent être préparés selon des méthodes qui y sont décrites ou qui sont connues de l'homme de métier.

Ainsi la 4-(5-méthyl-1*H*-imidazol-4-yl)pyridine est décrite dans *J. Med. Chem.*, 1986, **29**, 2154-63.

La 4-(1*H*-imidazol-4-yl)pipéridine est décrite dans *Arch. Pharmaz.*, (Weinheim. Ger.) 1973, **306**(12), 934-42 et dans la demande de brevet européen 0197840.

La 1,2,3,4-tétrahyroquinoléin-8-amine est décrite dans *Chemical Abstract*, 1939, **33**, 610.

La 5,6-dihydro-4*H*-imidazo[4,5,1-ij]quinoléin-2(1*H*)-one est préparée selon une méthode analogue à celle décrite dans *J. Org. Chem.*, 1960, **25**, 1138-47 et dans *Chemical Abstract*, 1965, **63**, 13272.

Le (2-hydroxy-1-méthyléthyl)carbamate de 1,1-diméthyléthyle est préparé selon la méthode décrite dans *Te-*

*trahedron Letters*, 1991, **47**(38), 8177-94.

La (*S*)-9-nitro-7-chloro-3-méthyl-3,4-dihydro-1*H*-1,4-benzodiazépin-2,5-dione est préparée selon la méthode décrite dans *J. Med. Chem.*, 1991, **34**(11), 3187-97.

La (*S*)-3-méthyl-2,3,4,5-tétrahydro-1*H*-1,4-benzodiazépin-9-amine est préparée selon la méthode décrite dans *J. Med. Chem.*, 1991, **34**(2), 746-51.

La (*S*)-5-méthyl-4,5,6,7-tétrahydroimidazo[4,5,1-*jk*][1,4]benzodiazépin-2(1*H*)-one est préparée selon une méthode analogue à celles décrites dans *J. Med. Chem.*, 1991, **34**(2), 747-51 et dans *J. Med. Chem.*, 1991, **34**(11), 3187-97.

Les exemples qui suivent illustrent en détail la préparation des composés selon l'invention.

Les microanalyses et les spectres IR et RMN confirment la structure des composés obtenus.

Les numéros des composés exemplifiés renvoient à ceux du tableau donné plus loin qui illustre les structures et les propriétés chimiques de quelques composés selon l'invention. Le rapport entre parenthèse correspond au rapport sel:base.

Exemple 1 (composé no 1)

2-[4-(1*H*-imidazol-4-yl)pipéridin-1-yl]-5,6-dihydro-4*H*-imidazo-[4,5,1-*ij*]quinoléine

1.1. 2-chloro-5,6-dihydro-4*H*-imidazo[4,5,1-*ij*]quinoléine

1.1.1. 1,2,3,4-tétrahyroquinoléin-8-amine

On dissout 25 g (0,169 mole) de quinoléin-8-amine dans 600 ml d'éthanol. On chauffe le milieu réactionnel à 70 °C et on ajoute 45 g de sodium par petits morceaux. On porte à la température de reflux jusqu'à disparition du sodium. Ensuite on évapore à sec, on reprend le résidu par un mélange éther:hexane (50:50), on filtre et on évapore de nouveau à sec.

On obtient 35 g de produit sous forme d'une huile que l'on utilise telle quelle dans l'étape suivante.

1.1.2. 5,6-dihydro-4*H*-imidazo[4,5,1-*ij*]quinoléin-2(1*H*)-one

A 32 g (0,216 mole) de 1,2,3,4-tétrahyroquinoléin-8-amine on ajoute 13 g (0,216 mole) d'urée et on chauffe le mélange pendant 3 heures à 180 °C. On ajoute de l'eau bouillante, on laisse refroidir le milieu réactionnel, on ajoute de l'éther et on agite pendant 2 heures. Ensuite on filtre le milieu réactionnel, on essore le précipité, on le lave successivement avec de l'eau et de l'éther et on le sèche sous vide.

On obtient 19 g de produit que l'on recristallise dans de l'isopropanol. On purifie le résidu par chromatographie sur colonne de gel de silice en éluant par un mélange dichlorométhane:méthanol (95:5).

On obtient 7 g de produit que l'on utilise tel quel dans l'étape suivante.

Point de fusion = 212 °C

1.1.3. 2-chloro-5,6-dihydro-4*H*-imidazo[4,5,1-*ij*]quinoléine

On chauffe à la température de reflux pendant 3 heures, 7,6 g (0,0436 mole) de 5,6-dihydro-4*H*-imidazo[4,5,1-*ij*]quinoléin-2(1*H*)-one en présence de 70 ml de chlorure de phosphoryle. On évapore le solvant à sec et on reprend le résidu par de la glace. On ajuste le pH du milieu réactionnel à 8 avec une solution de soude concentrée et on extrait avec du dichlorométhane. On sèche la phase organique sur sulfate de sodium et on évapore le solvant à sec. On purifie le résidu par chromatographie sur colonne de gel de silice en éluant par un mélange acétate d'éthyle:hexane (30:70).

On obtient 5,8 g de produit que l'on utilise tel quel dans l'étape suivante.

Point de fusion = 85-87 °C

1.2. 2-[4-(1*H*-imidazol-4-yl)piperidin-1-yl]-5,6-dihydro-4*H*-imidazo[4,5,1-*ij*]quinoléine

On chauffe à 120 °C pendant une journée, 1 g (0,0052 mole) de 2-chloro-5,6-dihydro-4*H*-imidazo[4,5,1-*ij*]quinoléine, 1,57 g (0,0104 mole) de 4-(1*H*-imidazol-4-yl)pipéridine et 5 ml d'alcool isoamylique. Ensuite on évapore l'alcool à sec, on reprend le résidu par un mélange eau:éther (50:50) et on essore le produit. On purifie le produit par chromatographie sur colonne de gel de silice en éluant par un mélange dichlorométhane:méthanol:ammoniaque (95:5:0,5) puis on le triture dans l'éther et on le sèche sous vide à 60 °C.

On obtient 1 g de produit.

Point de fusion = 250-255 °C

Exemple 2 (composé no 8)

(*Z*)-but-2-ènedioate de 4-méthyl-2-[4-(5-méthyl-1*H*-imidazol-4-yl)pipéridin-1-yl]-4,5-dihydroimidazo[1,5,4-*de*][1,4]benzoxazine (2:1)

2.1. 2-chloro-4-méthyl-4 5-dihydroimidazo[1,5,4-*de*] [1,4]benzoxazin-2-(1*H*)-one

2.1.1. (2-hydroxy-1-méthyléthyl)carbamate de 1,1-diméthyléthyle

On refroidit par un bain de glace une suspension de 7,5 g (0,1 mole) de 2-aminopropan-1-ol dans 100

ml d'une solution de soude 1 N et 100 ml de dichlorométhane et on ajoute petit à petit 22,8 g (0,105 mole) de dicarbonate de bis(1,1-diméthyléthyle) en solution dans 20 ml de dichlorométhane. On laisse la température du milieu réactionnel revenir à la température ambiante et on agite le mélange pendant une nuit. Après décantation on récupère la phase organique et on la lave deux fois avec de l'eau. On sèche et on évapore le solvant à sec.

On obtient 15 g de produit que l'on recristallise par trituration dans l'hexane.

2.1.2. 1-(2-chloro-3-nitrophénoxy)propan-2-amine

Dans un ballon tricol de 500 ml contenant 140 ml de benzène, on place 7,2 g (0,0414 mole) de (2-hydroxy-1-méthyléthyl)carbamate de 1,1-diméthyléthyle et 10,8 g (0,0414 mole) de triphénylphosphine. On refroidit le mélange au bain de glace et on ajoute goutte à goutte 6,5 ml (0,0414 mole) de diéthylazodicarboxylate. On laisse sous agitation pendant 15 minutes, on ajoute 4,8 g (0,027 mole) de 2-chloro-3-nitrophénol, on laisse la température du milieu réactionnel revenir à la température ambiante et on laisse sous agitation pendant une nuit. Ensuite on filtre le précipité obtenu et on évapore le filtrat. On reprend le résidu par 100 ml d'une solution d'acide chlorhydrique 3 N puis on chauffe à 80 °C au bain d'huile pendant 2 heures. On sépare les phases, on récupère la phase aqueuse, on ajuste à pH alcalin avec une solution de soude concentrée et on extrait trois fois avec de l'éther. On lave avec de l'eau et on sèche.

On obtient 6 g de produit que l'on utilise tel quel dans l'étape suivante.

2.1.3. 3-méthyl-5-nitro-3,4-dihydro-2*H*-1,4-benzoxazine

On chauffe à 110 °C sous agitation pendant une nuit 6 g (0,026 mole) de 1-(2-chloro-3-nitrophénoxy)propan-2-amine, 3,6 g (0,026 mole) de carbonate de potassium et 20 ml de *N,N*-diméthylformamide. On verse le milieu réactionnel sur de l'eau et on extrait 3 fois avec de l'éther. On récupère la phase organique, on la lave à l'eau, on la sèche et on évapore le solvant à sec.

On obtient 4 g de produit sous la forme d'un résidu cristallisé que l'on utilise tel quel dans l'étape suivante.

Point de fusion = 60 °C

2.1.4. 3-méthyl-3,4-dihydro-2*H*-1,4-benzoxazine-5-amine

On place dans un appareil de Parr 4 g (0,0205 mole) de 3-méthyl-5-nitro-3,4-dihydro-2*H*-1,4-benzoxazine en solution dans 100 ml d'éthanol. On réalise une hydrogénation catalytique en présence de palladium sur charbon à 5 % à la température ambiante sous une pression de 30 psi. On filtre le catalyseur, on le lave avec de l'éthanol, on récupère le filtrat et on évapore le solvant à sec.

On obtient 3,3 g de produit que l'on utilise tel quel dans l'étape suivante.

2.1.5. 4-méthyl-4,5-dihydroimidazo[1,5,4-*de*][1,4]benzoxazin-2(1*H*)-one

On chauffe à 160-165 °C au bain d'huile pendant 1,5 heures 3,3 g (0,02 mole) de 3-méthyl-3,4-dihydro-2*H*-1,4-benzoxazine-5-amine en présence de 1,7 g (0,028 mole) d'urée. On obtient un solide que l'on reprend par un mélange eau:éther (50:50). On sépare les phases, on récupère la phase organique, on la lave, on la sèche et on évapore le solvant à sec. On purifie le résidu obtenu par chromatographie sur colonne de gel de silice en éluant avec de l'éther éthylique.

On obtient 2 g de produit.

Point de fusion = 137 °C

2.1.6. 2-chloro-4-méthyl-4,5-dihydroimidazo[1,5,4-*de*][1,4] benzoxazine

On chauffe à la température de reflux pendant 2 heures, 1,9 g (0,01 mole) de 4-méthyl-4,5-dihydroimidazo[1,5,4-*de*][1,4]benzoxazin-2(1*H*)-one dans 35 ml de chlorure de phosphoryle. On évapore le solvant à sec et on reprend le résidu successivement par de l'eau glacée puis par une solution d'ammoniaque concentrée. Ensuite on extrait deux fois avec de l'éther, on réunit les phases organiques, on les sèche et on évapore à sec. On purifie le résidu par chromatographie sur colonne de gel de silice en éluant par un mélange acétate d'éthyle:hexane (40:60).

On obtient 1,5 g de produit sous forme d'une huile que l'on utilise telle quelle dans l'étape suivante.

2.2. (*Z*)-but-2-ènedioate de 4-méthyl-2-[4-(5-méthyl-1*H*-imidazol-4-yl)pipéridin-1-yl]-4,5-dihydroimidazo [1,5,4-*de*][1,4]benzoxazine (2:1)

On chauffe à 120 °C pendant 24 heures sous agitation 1,72 g (0,0072 mole) de 4-(5-méthyl-1*H*-imidazol-4-yl)pipéridine, 0,75 g (0,0036 mole) de 2-chloro-4-méthyl-4,5-dihydroimidazo[1,5,4-*de*][1,4]benzoxazine et 4 ml d'alcool isoamylique. Ensuite on évapore le solvant à sec et on reprend le résidu par un mélange eau:éther (50:50) puis on laisse sous agitation jusqu'à cristallisation du produit. On essore le produit cristallisé, on le lave successivement par de l'eau et par de l'éther puis on le purifie par chromatographie sur colonne de gel de silice en éluant par un mélange dichlorométhane:méthanol:ammoniaque (95:5:0,5). On recristallise le produit dans l'éther.

On prépare le bis maléate en ajoutant de l'acide maléique à la base dans un mélange alcool/éther éthylique.

Point de fusion = 140 °C

Exemple 3 (composé no 8a)

(*Z*)-but-2-ènedioate de (*S*)-4-méthyl-2-[4-(5-méthyl-1*H*-imidazol-4-yl)pipéridin-1-yl]-4,5-dihydroimidazo[1,5,4-*de*][1,4]benzoxazine (2:1)

3.1. (S)-2-chloro-4-méthyl-4,5-dihydroimidazo[1,5,4-*de*][1,4]benzoxazine

On obtient ce composé selon la méthode décrite dans l'exemple 2.1 à partir du (*S*)-2-aminopropan-1-ol.

3.2. (*Z*)-but-2-ènedioate de (*S*)-4-méthyl-2-[4-(5-méthyl-1*H*-imidazol-4-yl)pipéridin-1-yl]-4,5-dihydroimidazo [1,5,4-*de*][1,4]benzoxazine (2:1)

On prépare ce composé selon la méthode décrite dans l'exemple 2.2 à partir de la (*S*)-2-chloro-4-méthyl-4,5-dihydroimidazo [1,5,4-*de*][1,4]benzoxazine et de la 4-(5-méthyl-1*H*-imidazol-4-yl)pipéridine.

On obtient 2,1 g de composé sous forme de base qui cristallise dans un mélange dichlorométhane/éther.

$[\alpha]_D^{20}$ = -20,8 ° (c = 0,01; méthanol)

On prépare le maléate en ajoutant de l'acide maléique à la base dans un mélange méthanol/éther puis on sèche le produit à 100 °C.

Point de fusion = 136-140 °C

$[\alpha]_D^{20}$ = -8,3 ° (c = 0,01; méthanol)

Exemple 4 (composé no 8b)

(*Z*)-but-2-ènedioate de (*R*)-4-méthyl-2-[4-(5-méthyl-1*H*-imidazol-4-yl)pipéridin-1-yl]-4,5-dihydroimidazo[1,5,4-*de*][1,4]benzoxazine (2:1)

4.1. (*R*)-2-chloro-4-méthyl-4,5-dihydroimidazo[1,5,4-*de*] [1,4]benzoxazine

On obtient ce composé selon la méthode décrite dans l'exemple 2.1 à partir du (*R*)-2-aminopropan-1-ol.

4.2. (*Z*)-but-2-ènedioate de (*R*)-4-méthyl-2-[4-(5-méthyl-1*H*-imidazol-4-yl)pipéridin-1-yl]-4,5-dihydroimidazo[1,5,4-*de*][1,4]benzoxazine (2:1)

On obtient ce composé selon la méthode décrite dans l'exemple 2.2 à partir de la (*R*)-2-chloro-4-méthyl-4,5-dihydroimidazo [1,5,4-*de*][1,4]benzoxazine et de la 4-(5-méthyl-1*H*-imidazol-4-yl)pipéridine.

On obtient le composé sous forme de base qui cristallise dans un mélange dichlorométhane/éther.

Point de fusion = 143 °C

$[\alpha]_D^{20}$ = +22 ° (c = 0,01; méthanol)

On prépare le maléate en ajoutant de l'acide maléique à la base dans un mélange méthanol/éther puis on sèche le produit à 100 °C.

Point de fusion = 136-140 °C

$[\alpha]_D^{20}$ = +8,6 ° (c = 0,01; méthanol)

Exemple 5 (composé no 7a)

(*Z*)-but-2-ènedioate de (*S*)-4-méthyl-2-[4-(1*H*-imidazol-4-yl)pipéridin-1-yl]-4,5-dihydroimidazo[1,5,4-*de*][1,4]benzoxazine (2:1)

On obtient ce composé selon la méthode décrite dans l'exemple 2 à partir de la (*S*)-2-chloro-4-méthyl-4,5-dihydroimidazo [1,5,4-*de*][1,4]benzoxazine et de la 4-(1*H*-imidazol-4-yl)pipéridine.

On obtient le composé sous forme de base.

Point de fusion = 210-212 °C

$[\alpha]_D^{20}$ = -61,9 ° (c = 0,01; méthanol)

On prépare le maléate selon la méthode décrite dans l'exemple 2.

Point de fusion = 156 °C

$[\alpha]_D^{20}$ = -8,8 ° (c = 0,01; méthanol)

Exemple 6 (composé no 7b)

(*Z*)-but-2-ènedioate de (*R*)-4-méthyl-2-[4-(1*H*-imidazol-4-yl)pipéridin-1-yl]-4,5-dihydroimidazo[1,5,4-*de*][1,4]benzoxazine (2:1)

On obtient ce composé selon la méthode décrite dans l'exemple 2 à partir de la (*R*)-2-chloro-4-méthyl-4,5-dihydroimidazo [1,5,4-*de*][1,4]benzoxazine et de la 4-(1*H*-imidazol-4-yl)pipéridine.

On obtient le composé sous forme de base.
Point de fusion = 212 °C
$[\alpha]_D^{20}$ = +59,9 ° (c = 0,01; méthanol)
On prépare le maléate selon la méthode décrite dans l'exemple 2.
Point de fusion = 156 °C
$[\alpha]_D^{20}$ = +9,6 ° (c = 0,01; méthanol)

Exemple 7 (composé no 11a)

(*S*)-2-[4-(1*H*-imidazol-4-yl)pipéridin-1-yl]-4-phényl-4,5-dihydroimidazo[1,5,4-*de*][1,4]benzoxazine
On prépare ce composé selon le mode opératoire décrit dans l'exemple 2, à partir du (*S*)-(2-hydroxy-1-phényléthyl)carbamate de 1,1-diméthyléthyle et de la 4-(1*H*-imidazol-4-yl) pipéridine.
On obtient le produit sous forme de base.
Point de fusion = 135-140 °C
$[\alpha]_D^{20}$ = +108,2 ° (c = 0,01; méthanol)

Exemple 8 (composé no 11b)

(*R*)-2-[4-(1*H*-imidazol-4-yl)pipéridin-1-yl]-4-phényl-4,5-dihydroimidazo[1,5,4-*de*][1,4]benzoxazine
On prépare se composé selon le mode opératoire décrit dans l'exemple 2, à partir du (*R*)-(2-hydroxy-1-phényléthyl)carbamate de 1,1-diméthyléthyle et de la 4-(1*H*-imidazol-4-yl) pipéridine.
On obtient le produit sous forme de base.
Point de fusion = 135-140 °C
$[\alpha]_D^{20}$ = -114 ° (c = 0,01; méthanol)

Exemple 9 (composé no 12a)

(*S*)-2-[4-(5-méthyl-1*H*-imidazol-4-yl)pipéridin-1-yl]-4-phényl-4,5-dihydroimidazo[1,5,4-*de*][1,4]benzoxazine
On obtient ce composé selon le mode opératoire décrit dans l'exemple 2, à partir du (*S*)-(2-hydroxy-1-phényléthyl)carbamate de 1,1-diméthyléthyle et de la 4-(5-méthyl-1*H*-imidazol-4-yl)pipéridine.
On obtient le produit sous forme de base.
Point de fusion = 206 °C
$[\alpha]_D^{20}$ = +95,2 ° (c = 0,01; méthanol)

Exemple 10 (composé no 12b)

(*R*)-2-[4-(5-méthyl-1*H*-imidazol-4-yl)pipéridin-1-yl]-4-phényl-4,5-dihydroimidazo[1,5,4-*de*][1,4]benzoxazine
On obtient ce composé selon le mode opératoire décrit dans l'exemple 2, à partir du (*R*)-(2-hydroxy-1-phényléthyl)carbamate de 1,1-diméthyléthyle et de la 4-(5-méthyl-1*H*-imidazol-4-yl)pipéridine.
On obtient le produit sous forme de base.
Point de fusion = 206 °C
$[\alpha]_D^{20}$ = -98,1 ° (c = 0,01; méthanol)

Exemple 11 (composé no 14)

éthanedioate de 2-[4-(1*H*-imidazol-4-yl)pipéridin-1-yl]-5,6-dihydro-4*H*-imidazo[1,5,4-*de*]quinazoline (1,5:1)

11.1. 2-chloro-5,6-dihydro-4*H*-imidazo[1,5,4-*de*]quinazoline

11.1.1. 2-[(2,6-dinitrophényl)amino]éthanol

Dans un ballon tricol contenant 180 ml d'éthanol, on place 25 g (0,123 mole) de 1-chloro-2,6-dinitrobenzène. On chauffe le milieu réactionnel à 70 °C, on ajoute goutte à goutte en 15 minutes, 22,3 g (0,365 mole) d'éthanolamine et on laisse le mélange sous agitation à cette température pendant 30 minutes. On laisse la température du milieu réactionnel revenir à la température ambiante puis on ajoute 1 litre d'eau. Le produit cristallise, on l'essore, on le lave à l'eau et on le sèche.
On obtient 25 g de produit que l'on utilise tel quel dans l'étape suivante.
Point de fusion = 77 °C

11.1.2. dichlorhydrate de 2-[(2,6-diaminophényl)amino]éthanol

On chauffe à 80 °C au bain d'huile pendant 15 minutes, 18,3 g (0,0805 mole) de 2-[(2,6-dinitrophényl)amino]éthanol dans une solution contenant 144 g de chlorure stanneux dihydraté dans 146 ml d'une solution d'acide chlorhydrique concentré. On refroidit le milieu réactionnel au bain de glace, on ajoute lentement 460 ml d'une solution de soude concentrée puis on extrait trois fois par du dichlorométhane. On réunit les phases organiques, on les sèche et on évapore à sec.

On obtient le produit sous forme de base.

On prépare le chlorhydrate dans un mélange alcool/éther chlorhydrique.

On obtient le chlorhydrate que l'on utilise tel quel dans l'étape suivante.

Point de fusion = 195-200 °C

11.1.3. <u>1,2,3,4-tétrahydroquinazolin-5-amine</u>

On chauffe à 160 °C pendant 3 heures, 25 g (0,104 mole) de dichlorhydrate de 2-[(2,6-diaminophényl)amino]éthanol dans 300 ml d'une solution d'acide bromhydrique à 62 %. On refroidit le milieu réactionnel à -20 °C jusqu'à cristallisation et on sèche le précipité obtenu, sous azote, avec un mélange de méthanol et d'éther. On le reprend par de l'eau, on ajuste à pH alcalin avec une solution de soude concentrée et on extrait par un mélange dichlorométhane:éther (50:50). On sèche et on évapore.

On obtient 11,5 g de produit que l'on utilise tel quel dans l'étape suivante.

11.1.4. <u>5,6-dihydro-4*H*-imidazo[1,5,4-*de*]quinazolin-2(1*H*)-one</u>

On chauffe à 160-165 °C au bain d'huile pendant 2 heures 11,5 g (0,078 mole) de 1,2,3,4-tétrahydroquinazolin-5-amine en présence de 6,7 g (0,028 mole) d'urée. On obtient un solide que l'on reprend avec 20 ml d'eau. On refroidit le mélange au bain de glace, on essore, on lave à l'eau et on sèche le précipité obtenu. On purifie le résidu par chromatographie sur colonne de gel de silice en éluant par un mélange dichlorométhane/méthanol/ammoniaque (95/5/0,5).

On obtient 5,5 g de produit qui cristallise dans l'éthanol.

Point de fusion = 206 °C

11.1.5. <u>2-chloro-5,6-dihydro-4*H*-imidazo[1,5,4-*de*]quinazoline</u>

On chauffe à la température de reflux pendant 3 heures 3 g (0,0155 mole) de 5,6-dihydro-4*H*-imidazo[1,5,4-*de*]quinazolin-2(1*H*)-one dans 60 ml de chlorure de phosphoryle. On évapore le solvant à sec et on reprend le résidu successivement par de l'eau glacée et par une solution d'ammoniaque concentrée. Ensuite on extrait deux fois avec de l'éther, on réunit les phases organiques, on sèche et on évapore à sec. On purifie le résidu par chromatographie sur colonne de gel de silice en éluant par de l'éther.

On obtient 1,8 g de produit sous forme d'huile que l'on utilise telle quelle dans l'étape suivante.

11.2. <u>éthanedioate de 2-[4-(1*H*-imidazol-4-yl)pipéridin-1-yl]-5,6-dihydro-4*H*-imidazo[1,5,4-*de*]quinazoline (1,5:1)</u>

On procède selon la méthode décrite dans l'exemple 1.2, à partir de la 2-chloro-5,6-dihydro-4*H*-imidazo[1,5,4-*de*]quinazoline et de la 4-(1*H*-imidazol-4-yl)pipéridine.

On obtient 0,95 g de produit sous forme de base.

On la recristallise dans un mélange alcool/éther, on la dissout dans du méthanol et on prépare l'oxalate.

Point de fusion = 212 °C

<u>Exemple 12</u> (composé no 19)

<u>(*E*)-but-2-ènedioate de (*S*)-5-méthyl-2-[4-(5-méthyl-1*H*-imidazol-4-yl)pipéridin-1-yl]-4,5,6,7-tétrahydroimidazo[4,5,1-*jk*][1,4]benzodiazépine (2:1)</u>

12.1. <u>(*S*)-2-chloro-5-méthyl-4,5,6,7-tétrahydroimidazo [4,5,1-*jk*][1,4]benzodiazépine</u>

12.1.1. <u>(*S*)-9-amino-3-méthyl-3,4-dihydro-1*H*-1,4-benzodiazépin-2,5-dione</u>

Dans une bouteille de Parr, on place 6 g (0,022 mole) de (*S*)-7-chloro-3-méthyl-9-nitro-3,4-dihydro-1*H*-1,4-benzodiazépin-2,5-dione, 200 ml d'eau et 200 ml d'acide acétique et 0,5 g de palladium sur charbon à 5 %. On réalise une hydrogénation catalytique sous 40 psi à 50 °C. On filtre le catalyseur, on le lave avec un mélange acide acétique:eau (50:50) et on concentre le filtrat à sec. On le reprend par de l'eau et on ajuste à pH alcalin avec une solution de carbonate de sodium. On laisse sous agitation pendant 15 minutes, on filtre, on sèche et on évapore le solvant à sec.

On obtient 4 g de produit.

Point de fusion = 320 °C

12.1.2. <u>(*S*)-3-méthyl-2,3,4,5-tétrahydro-1*H*-1,4-benzodiazépin-9-amine</u>

On chauffe à la température de reflux sous agitation pendant 48 heures, 14,8 g (0,072 mole) de (*S*)-9-amino-3-méthyl-3,4-dihydro-1*H*-1,4-benzodiazépin-2,5-dione dans 455 ml de dioxane en présence de 17 g d'hydrure de lithium et d'aluminium. On refroidit le milieu réactionnel au bain de glace puis on ajoute lentement et successivement 17 ml d'eau, 17 ml d'une solution de soude 5 N et 45 ml d'eau. On

laisse sous agitation pendant 2 heures à la température ambiante, on filtre et on lave avec du tétrahydrofurane chaud et du dichlorométhane.

On récupère la phase organique et on la concentre.

On obtient 12,7 g de produit que l'on utilise tel quel dans l'étape suivante.

12.1.3. (*S*)-5-méthyl-4,5,6,7-tétrahydroimidazo[4,5,1-*jk*] [1,4]benzodiazépin-2(1*H*)-one

A 12,7 g (0,072 mole) de (*S*)-3-méthyl-2,3,4,5-tétrahydro-1*H*-1,4-benzodiazépin-9-amine on ajoute 300 ml de dichlorométhane et 22,8 ml de 4-méthylmorpholine. Ensuite on verse le mélange sur une solution de 9,1 ml (0,0072 mole) de chloroformiate de trichlorométhyle dans 340 ml de dichlorométhane préalablement refroidie par un bain de glace. On laisse sous agitation pendant 10 minutes à 0 °C puis pendant 20 minutes à la température ambiante. On évapore le solvant sous vide et on reprend le résidu dans 200 ml d'eau et 34 ml de dioxane. On chauffe le mélange au bain marie pendant 45 minutes puis on ajoute une solution d'ammoniaque concentrée. On laisse refroidir le mélange, on l'essore, on le lave à l'eau et on le sèche sous vide. On obtient 11,5 g de produit que l'on recristallise dans de l'eau bouillante.

On obtient 7,6 g de produit que l'on utilise tel quel dans l'étape suivante.

Point de fusion = 198-203 °C

12.1.4. (*S*)-2-chloro-5-méthyl-4,5,6,7-tétrahydroimidazo [4,5,1-*jk*][1,4]benzodiazépine

On chauffe à 130 °C au bain d'huile pendant 4 heures 4,55 g (0,022 mole) de (*S*)-5-méthyl-4,5,6,7-tétrahydroimidazo [4,5,1-*jk*][1,4]benzodiazépin-2(1*H*)-one dans 90 ml de chlorure de phosphoryle. On évapore le solvant sous vide et on reprend à chaud le résidu huileux, par de l'eau. On laisse le mélange sous agitation pendant 15 minutes, on le refroidit et on ajoute une solution d'ammoniaque concentrée. On extrait par du dichlorométhane, on lave à l'eau, on sèche sur sulfate de sodium et on évapore le solvant à sec.

On obtient 4,3 g de produit sous forme d'une huile que l'on utilise telle quelle dans l'étape suivante.

12.2. (*E*)-but-2-ènedioate de (*S*)-5-méthyl-2-[4-(5-méthyl-1*H*-imidazol-4-yl)pipéridin-1-yl]-4,5,6,7-tétrahydroimidazo[4,5,1-*jk*][1,4]benzodiazépine (2:1)

On chauffe à 120 °C au bain d'huile pendant 15 heures, 2,36 g (0,0072 mole) de 4-(5-méthyl-1*H*-imidazol-4-yl)pipéridine et 1,5 g (0,0071 mole) de (*S*)-2-chloro-5-méthyl-4,5,6,7-tétrahydroimidazo[4,5,1-*jk*][1,4]benzodiazépine dans 8 ml d'alcool isoamylique. Ensuite on évapore le solvant et on purifie le résidu par chromatographie sur colonne de gel de silice en éluant par un mélange dichlorométhane:méthanol:ammoniaque (95:5:0,5). On recueille les fractions pures, on les concentre sous vide et on cristallise le produit dans de l'acétone.

On obtient 0,4 g de produit sous forme de base.

On prépare le bis fumarate en ajoutant de l'acide fumarique à la base dans l'éthanol. On le recristallise dans du méthanol.

On obtient 0,4 g de produit sous forme de bisfumarate.

Point de fusion = 196-198 °C

$[\alpha]_D^{20}$ = +1 ° (c = 0,01; méthanol)

Exemple 13 (composé no 18)

(*Z*)-but-2-ènedioate de (*S*)-2-[4-(1*H*-imidazol-4-yl)pipéridin-1-yl]-5-méthyl-4,5,6,7-tétrahydroimidazo[4,5,1-*jk*][1,4]benzodiazépine (3:1)

On chauffe à 120 °C au bain d'huile pendant 12 heures, 4,9 g (0,0324 mole) de 4-(1*H*-imidazol-4-yl)pipéridine et 4,3 g (0,0193 mole) de (*S*)-2-chloro-5-méthyl-4,5,6,7-tétrahydro-imidazo[4,5,1-*jk*][1,4]benzodiazépine dans 15 ml d'alcool isoamylique. Ensuite on évapore le solvant et on purifie le résidu par chromatographie sur colonne de gel de silice en éluant par un mélange dichlorométhane:méthanol:ammoniaque (93:7:0,7). On recueille les fractions pures, on les concentre sous vide et on cristallise le produit dans 15 ml d'acétone bouillant. On l'essore, on le lave avec de l'acétone et on le sèche.

On obtient 2,8 g de produit sous forme de base.

On prépare le maléate en ajoutant de l'acide maléique à la base dans un mélange alcool/éther. On recristallise le produit sous forme de sel dans un mélange méthanol/éther.

Point de fusion = 132-134 °C

$[\alpha]_D^{20}$ = +2 ° (0,005; méthanol)

Exemple 14 (composé no 20)

(*Z*)-but-2-ènedioate de (*S*)-2-[4-(1*H*-imidazol-4-yl)pipéridin-1-yl]-5-méthyl-6-(phénylméthyl)-4,5,6,7-tétrahydroimidazo[4,5,1-*jk*][1,4]benzodiazépine (2:1)

A une suspension de 0,45 g (0,0013 mole) de (*S*)-2-[4-(1*H*-imidazol-4-yl)pipéridin-1-yl]-5-méthyl-4,5,6,7-tétrahydroimida-zo[4,5,1-*jk*][1,4]benzodiazépine dans 4,5 ml d'éthanol contenant 0,035 g (0,00253 mole) de carbonate de potassium, on ajoute 0,16 g (0,00134 mole) de (bromométhyl)benzène. On chauffe le mélange à 60 °C au bain d'huile pendant 2 heures puis on évapore le solvant à sec. On purifie le résidu par chromatographie sur colonne de gel de silice en éluant par un mélange dichlorométhane:méthanol:ammoniaque (95:5:0,5).

On récupère 0,3 g de produit sous forme de base.

On prépare le bis maléate en ajoutant de l'acide maléique à la base dans l'acétone. On l'essore, on le lave à l'acétone et on le sèche.

Point de fusion = 160-162 °C

$[\alpha]_D^{20}$ = +1,1 ° (c = 0,01; méthanol)

Tableau

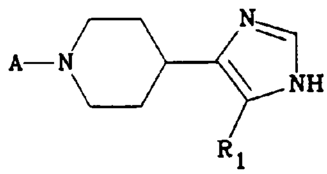

(I)

| No | A | $R_1$ | F (°C) | Sel | $[\alpha]_D^{20}$ (°) |
|---|---|---|---|---|---|
| 1 | 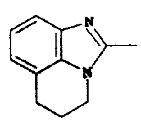 | -H | 250-255 | - | - |
| 2 | 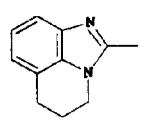 | $-CH_3$ | 231-233 | - | - |
| 3 | 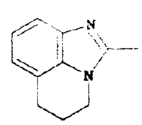 | $-CH_2CH_3$ | 219-221 | - | - |
| 4 | 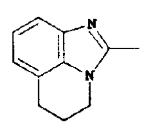 | $-CH(CH_3)_2$ | 175-177 | - | - |

| No | A | $R_1$ | F (°C) | Sel | $[\alpha]_D^{20}$ (°) |
|---|---|---|---|---|---|
| 5 | 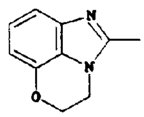 | -H | 172 | mal. (2:1) | - |
| 6 | 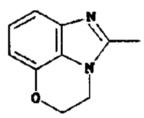 | $-CH_3$ | 150 | mal. (2:1) | - |
| 7 | 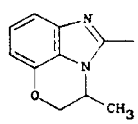 | -H | 148 | mal. (2:1) | - |
| 7a | 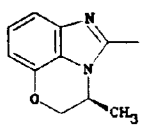 | -H | 156 | mal. (2:1) | - 8,8 |
| 7b | 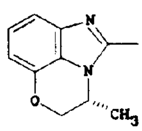 | -H | 156 | mal. (2:1) | + 9,6 |
| 8 | 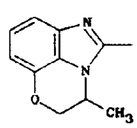 | $-CH_3$ | 140 | mal. (2:1) | - |
| 8a | 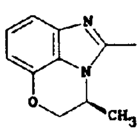 | $-CH_3$ | 136-140 | mal. (2:1) | - 8,3 |

| No | A | $R_1$ | F (°C) | Sel | $[\alpha]_D^{20}$ (°) |
|---|---|---|---|---|---|
| 8b | 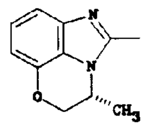 | $-CH_3$ | 136-140 | mal. (2:1) | + 8,6 |
| 9 | 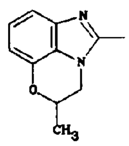 | -H | 184-185 | mal. (2:1) | - |
| 10 | 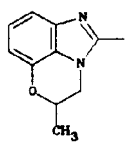 | $-CH_3$ | 239-241 | ox. (2:1) | - |
| 11a | 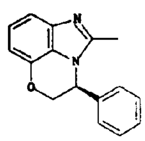 | -H | 135-140 | - | + 108,2 |
| 11b | 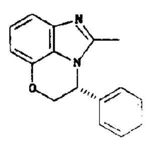 | -H | 135-140 | - | - 114 |
| 12a | 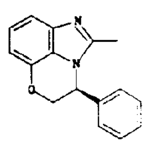 | $-CH_3$ | 206 | - | + 95,2 |
| 12b | 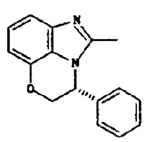 | $-CH_3$ | 207 | - | - 98,1 |

| No | A | $R_1$ | F (°C) | Sel | $[\alpha]_D^{20}$ (°) |
|---|---|---|---|---|---|
| 13 | 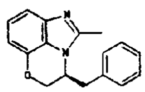 | $-CH_3$ | 210-212 | fum. (1:1) | - 59,9 |
| 14 | 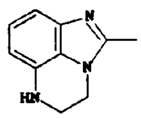 | -H | 212 | ox. (1,5:1) | - |
| 15 | 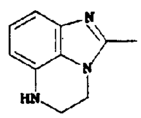 | $-CH_3$ | 212 | ox. (1:1) | - |
| 16 | 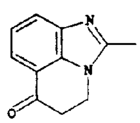 | -H | 200 | - | - |
| 17 | 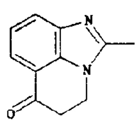 | $-CH_3$ | 130 | - | - |
| 18 | 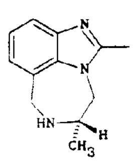 | -H | 132-134 | mal. (3:1) | + 2 |
| 19 | 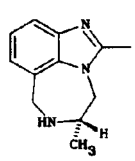 | $-CH_3$ | 196-198 | fum. (2:1) | + 1 |

| No | A | $R_1$ | F (°C) | Sel | $[\alpha]_D^{20}$ (°) |
|---|---|---|---|---|---|
| 20 | 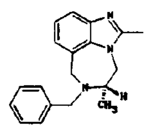 | -H | 160-162 | mal. (2:1) | + 1,1 |

**Légende du tableau :**

dans la colonne "Sel" du tableau

(x:y) signifie x moles d'acide pour y moles de base, l'absence de toute mention signifie que le composé est à l'état de base,

mal. représente le maléate,
fum. représente le fumarate
ox. représente l'oxalate

dans la colonne "$[\alpha]_D^{20}$" du tableau

c = 0,01; méthanol sauf pour le composé no 18 où c = 0,005
l'absence de toute mention signifie que le composé est un racémate.

Les composés de l'invention ont fait l'objet d'essais pharmacologiques qui ont montré leur intérêt comme substances actives en thérapeutique.

Ainsi ils ont été testés quant à leurs effets inhibiteurs de la liaison de la [$^3$H]quipazine avec les récepteurs sérotoninergiques de type 5-$HT_3$ présents dans le cortex cérébral du rat, selon une variante de la méthode décrite par Milburn et Peroutka (*J. Neurochem.*, **52**, 1787-1792, 1989).

On utilise des rats mâles Sprague-Dawley, de 150 à 200 g, dans tous les essais. On en prélève le cortex cérébral et on l'homogénéise dans 20 volumes (poids/volume) de tampon Hepes 25 mM ou de tampon Hepes 25 mM contenant du chlorure de sodium (180 mM), du chlorure de calcium (2,5 mM), du chlorure de potassium (5 mM) et du chlorure de magnésium (1,2 mM) (pH=7,4), à l'aide d'un broyeur Polytron™. Après centrifugation de la suspension pendant 10mn à 45000xg, on remet le culot en suspension dans le volume initial de tampon, contenant éventuellement 0,05% de Triton X-100™, et on effectue une première incubation de 30mn à 37°C. On effectue ensuite encore deux centrifugations comme précédemment décrit, et on reprend le culot final dans 11,7 volumes de tampon Hepes 25 mM à pH=7,4.

On détermine la liaison de la [$^3$H]quipazine (51,6-69,8 Ci/mmole, New England Nuclear, Boston, Ma, USA) en faisant incuber 150 µl de la suspension membranaire avec le radioligand (0,8 nM) dans un volume final de 1 ml, pendant 30mn à 25°C, en absence ou en présence du composé à étudier. L'incubation a lieu en présence de 0,1 µM de paroxétine et de 1 µM de kétansérine. La liaison non spécifique est déterminée en présence de 1 µM d'ondansetron. Après l'incubation, on dilue le mélange testé avec 5 ml de tampon Tris-HCl glacé 50 mM (pH=7,4 à 0°C). On collecte les membranes par filtration sur des filtres Whatman GF/B™ prétraités avec 0,05% de polyéthylèneimine, et on les lave avec trois volumes de 5 ml de tampon Tris-HCl glacé 50 mM.

La radioactivité retenue sur les filtres est mesurée par spectrométrie de scintillation liquide à une efficacité de 50 à 60%.

Les résultats s'expriment par la concentration ($CI_{50}$) de composé étudié qui inhibe 50% de la liaison de la [$^3$H]quipazine, déterminée par une méthode graphique ou mathématique. Les composés de l'invention les plus

actifs dans cet essai se caractérisent par des $CI_{50}$ comprises entre 0,01 nM et 10 nM.

Les composés de l'invention ont été également testés quant à leur effet sur le réflexe de Bezold-Jarisch, c'est-à-dire une intense bradycardie, provoqué par injection intraveineuse de sérotonine. Ce reflexe met en jeu la stimulation des récepteurs spécifiques 5-$HT_3$ du nerf vague, ce qui provoque une dépolarisation et donc une sécrétion d'acétylcholine, qui est le neurotransmetteur vagal naturel.

On anesthésie des rats mâles Sprague-Dawley à l'uréthane (1 à 25 g/kg par voie intrapéritonéale), on mesure la pression sanguine grâce à un cathéter placé dans l'artère carotide, et on se sert des impulsions de pression pour activer un cardiotachymètre. Des canules sont placées dans les deux veines fémorales pour faciliter l'administration intraveineuse des produits.

On trace les courbes doses/réponses de la bradycardie provoquée par injection de doses de 30 µg/kg de sérotonine, avant et après l'injection des composés à étudier.

Les composés de l'invention inhibent la bradycardie provoquée par la sérotonine de 75 % à la dose de 1 µg/kg administrée par voie intraveineuse.

Les composés de l'invention ont été également étudiés quant à leur affinité vis à vis des récepteurs 5-$HT_4$ dans le striatum de cobaye selon la méthode décrite par Grossman et coll. *Br. J. Pharmacol.*, (1993), 109, 618-624.

On euthanasie des cobayes (Hartley, Charles River) de 300 à 400 g et on prélève le cerveau. On excise les striata et on les congèle à - 80 °C. Le jour de l'expérience, on décongèle le tissu à + 4 °C dans 33 volumes de tampon Hépès-NaOH 50 mM (pH 7,4 à 20 °C) et on l'homogénéise à l'aide d'un broyeur Polytron ™. On centrifuge l'homogénat pendant 10 minutes à 48000 g, on récupère le culot, on le remet en suspension et on le centrifuge de nouveau dans les mêmes conditions. On suspend le culot final dans du tampon Hépès-NaOH (30 mg de tissu frais/ml). Cette suspension membranaire est utilisée telle quelle.

On incube 100 µl de la suspension membranaire à 0 °C pendant 120 minutes, en présence de 0,1 nM de $[^3H]$GR118808 (activité spécifique 80-85 Ci/mmoles), dans un volume final de 1 ml de tampon Hépès-NaOH (50 mM, pH 7,4), en présence ou en absence de composés à tester. On arrête l'incubation par filtration sur filtre Whatman GF/B, préalablement traités avec du polyéthylèneimine 0,1 %, on rince chaque tube par 4 ml de tampon à 0 °C et on filtre de nouveau. On mesure la radioactivité retenue sur les filtres par scintigraphie liquide.

On détermine la liaison non spécifique en présence de sérotonine 30 µM.

La liaison spécifique représente 90% de la radioactivité totale recupérée sur le filtre.

Pour chaque concentration de composé étudié, on détermine le pourcentage d'inhibition de la liaison spécifique du $[^3H]$GR118808 puis la concentration du composé testé qui inhibe 50% de la liaison spécifique ($CI_{50}$). Les $CI_{50}$ des composés selon l'invention se situent entre 0,02 et 2 µM.

Les résultats des essais biologiques montrent que les composés de l'invention sont des ligands des récepteurs sérotoninergiques de types 5-$HT_3$ et 5-$HT_4$.

Ils peuvent donc être utilisés pour le traitement et la prévention des désordres dans lesquels les récepteurs 5-$HT_3$ et 5-$HT_4$ sont impliqués, tels que nausées et vomissements, par exemple consécutifs à un traitement antitumoral ou à l'administration d'un anesthésique ; troubles du système nerveux central tels que la schizophrénie, la manie, l'anxiété et la dépression ; troubles de la cognition tels que la démence sénile ou présénile d'Alzheimer ; dyskinésie, douleurs, migraines et maux de tête ; troubles de la dépendance ou du sevrage d'alcool ou de drogues ; troubles de la fonction gastrointestinale tels que dyspepsie, ulcère peptique, aigreurs d'estomac, flatulences ; troubles du système cardiovasculaire et troubles respiratoires.

Ils peuvent également être utilisés pour le traitement et la prévention des désordres tels que la diarrhée, le colon irritable, le reflux oesophagien, les troubles moteurs intestinaux, les troubles de la sécrètion intestinale, la fibrose kystique du pancréas, le syndrome carcinoïde et l'incontinence.

A cet effet ils peuvent être présentés sous toutes formes appropriées à l'administration orale ou parentérale, telles que comprimés, dragées, gélules, capsules, suspensions ou solutions buvables ou injectables, etc. en association avec des excipients convenables, et dosées pour permettre une administration de 0,005 à 5 mg/kg de 1 à 4 fois par jour.

## Revendications

1. Composés répondant à la formule (I)

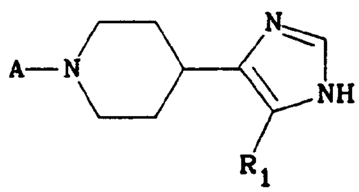

(I)

dans laquelle
$R_1$ représente soit un atome d'hydrogène, soit un groupe $(C_1-C_6)$alkyle droit ou ramifié et
A représente soit un groupe 5,6-dihydro-4*H*-imidazo[4,5,1-*ij*] quinoléin-2-yle, soit un groupe 4,5-dihydroimidazo[1,5,4-*de*] [1,4]benzoxazin-2-yle, soit un groupe 4-méthyl-4,5-dihydro-imidazo[1,5,4-*de*][1,4]benzoxazin-2-yle, soit un groupe 4-phényl-4,5-dihydroimidazo[1,5,4-*de*][1,4]benzoxazin-2-yle, soit un groupe 4-(phénylméthyl)-4,5-dihydroimidazo[1,5,4-*de*] [1,4]benzoxazin-2-yle, soit un groupe 5-méthyl-4,5-dihydroimidazo[1,5,4-*de*][1,4]benzoxazin-2-yle, soit un groupe 5,6-dihydro-4*H*-imidazo[1,5,4-*de*]quinazolin-2-yle, soit un groupe 6-oxo-5,6-dihydro-4*H*-imidazo[4,5,1-*ij*]quinoléin-2-yle, soit un groupe 5-méthyl-4,5,6,7-tétrahydroimidazo[4,5,1-*jk*] [1,4]benzodiazépin-2-yle éventuellement substitué en 6 par un groupe phénylméthyle
ainsi que leurs sels d'additions aux acides pharmaceutiquement acceptables.

**2.** Procédé de préparation des composés selon la revendication 1, procédé caractérisé en ce que l'on fait réagir à chaud dans un solvant un dérivé de formule (II)

A-Z (II)

dans laquelle
Z représente un atome d'halogène, de préférence un atome de chlore et
A est tel que défini dans la revendication 1
avec une pipéridine de formule (III)

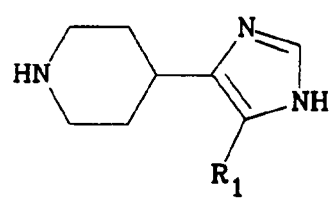

(III)

dans laquelle $R_1$ est tel que défini dans la revendication 1.

**3.** Médicament caractérisé en ce qu'il contient un composé selon la revendication 1.

**4.** Composition pharmaceutique caractérisée en ce qu'elle contient un composé selon la revendication 1 associé à tout excipient pharmaceutiquement acceptable.

Office européen des brevets

# RAPPORT DE RECHERCHE EUROPEENNE

Numero de la demande
EP 94 40 2114

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| D,A | EP-A-0 507 650 (SYNTHELABO) 7 Octobre 1992<br>* page 10, ligne 21 - ligne 30; revendication 1 *<br>----- | 1,3 | C07D471/06<br>C07D498/06<br>C07D487/06<br>A61K31/445<br>A61K31/535<br>A61K31/55<br>//(C07D471/06, 235:00, 221:00), (C07D498/06, 265:00, 235:00), (C07D487/06, 243:00, 235:00), (C07D487/06, 241:00,235:00) |
| | | | DOMAINES TECHNIQUES RECHERCHES (Int.Cl.6) |
| | | | C07D<br>A61K |

Le présent rapport a été établi pour toutes les revendications

| Lieu de la recherche | Date d'achèvement de la recherche | Examinateur |
|---|---|---|
| LA HAYE | 13 Décembre 1994 | Alfaro Faus, I |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)